Europäisches Patentamt

European Patent Office

Office européen des brevets

(11) Veröffentlichungsnummer: **0 007 602**
A1

# (12) EUROPÄISCHE PATENTANMELDUNG

(21) Anmeldenummer: 79102583.6

(22) Anmeldetag: 21.07.79

(51) Int. Cl.³: **A 61 M 1/00**

(30) Priorität: 25.07.78 DE 7822205 U

(43) Veröffentlichungstag der Anmeldung:
06.02.80 Patentblatt 80/3

(84) Benannte Vertragsstaaten:
AT BE CH DE FR GB IT LU NL SE

(71) Anmelder: Intermedicat GmbH
Gerliswilstrasse 45
CH-6020 Emmenbrücke(CH)

(72) Erfinder: Herlitze, Gerhard
Binsdorfer Strasse 2
D-3507 Baunatal(DE)

(74) Vertreter: von Kreisler, Alek, Dipl.-Chem. et al,
Deichmannhaus am Hauptbahnhof
D-5000 Köln 1(DE)

(54) Medizinisches Absaugrohr.

(57) Medizinisches Absaugrohr zum Absaugen von Körperprodukten oder Spülflüssigkeiten bei Operationen mit einem Mundstück (1) an einem Ende und einer Saugvorrichtung am anderen Ende (4), wobei das Rohr (6) auf einem Teilstück seiner Länge eine sich axial erstreckende Wanddurchbrechung (5) aufweist und auf dem Rohr eine Hülse beweglich angeordnet ist.

Fig. 1

EP 0 007 602 A1

Croydon Printing Company Ltd

## Medizinisches Absaugrohr

Die vorliegende Erfindung betrifft medizinische
Absaugrohre zum Absaugen von Körperprodukten
oder Spülflüssigkeiten im Verlaufe einer Operation.

Die gebräuchlichsten Absaugrohre bestehen aus
einem abgewinkelten Kunststoffrohr mit angeformter Öffnung oder mit einem Mundstück auf
der einen Seite, welches beim Absaugen mit
der jeweiligen Partie des Patienten in Kontakt
gebracht wird und einem Schlauchansatz auf der
anderen Seite, der einen Verbindungsschlauch
zu einer Vakuumquelle aufnimmt.

Da die Absaugrohre im allgemeinen verhältnismäßig dünn sind, kann zur leichteren Handhabung koaxial zu dem Rohr noch ein im Durchmesser größerer Handgriff angebracht sein.
Zusätzlich kann eine radiale Bohrung in der
Rohrwand vorhanden sein, die mit dem Daumen
oder Zeigefinger zur Sogregulierung verschlossen werden kann; läßt man die Bohrung
offen, so wird hier Nebenluft angesaugt und
am Mundstück kann sich kein wirksamer Unterdruck aufbauen. Derartige Absaugrohre sind für
einmaligen Gebrauch bestimmt.

Mit der radialen Bohrung zur Sogregulierung
kann praktisch nur eine Auf – Zu Funktion
realisiert werden. Es ist sehr schwierig
und würde sehr große Aufmerksamkeit des
medizinischen Personals erfordern, wenn
durch teilweises Abdecken der Bohrung ein
manchmal erwünschter mittlerer Unterdruck
am Saugmund erzeugt werden soll.

- 2 -

Aus dem genannten Nachteil der bekannten Absaugrohr-Ausführungen ergibt sich folgende Aufgabe:

- Schaffung einer Einrichtung zur Unterdruckbegrenzung, die zusätzlich zu der Auf - Zu-Funktion der Bohrung zur Sogregulierung noch die Einstellung von Druck-Zwischenstufen ermöglicht.

Gelöst wird die Aufgabe durch ein medizinisches Absaugrohr zum Absaugen von Körperprodukten oder Spülflüssigkeiten bei Operationen, dessen eines Ende ein Mundstück trägt und dessen anderes Ende an eine Saugvorrichtung angeschlossen ist, das dadurch gekennzeichnet ist, daß das Rohr (2) auf einem Teilstück seiner Länge eine sich axial erstreckende Wanddurchbrechung aufweist und auf dem Rohr eine Hülse (6) beweglich angeordnet ist.

Weitere Ausführungsformen sind in den Unteransprüchen beschrieben.

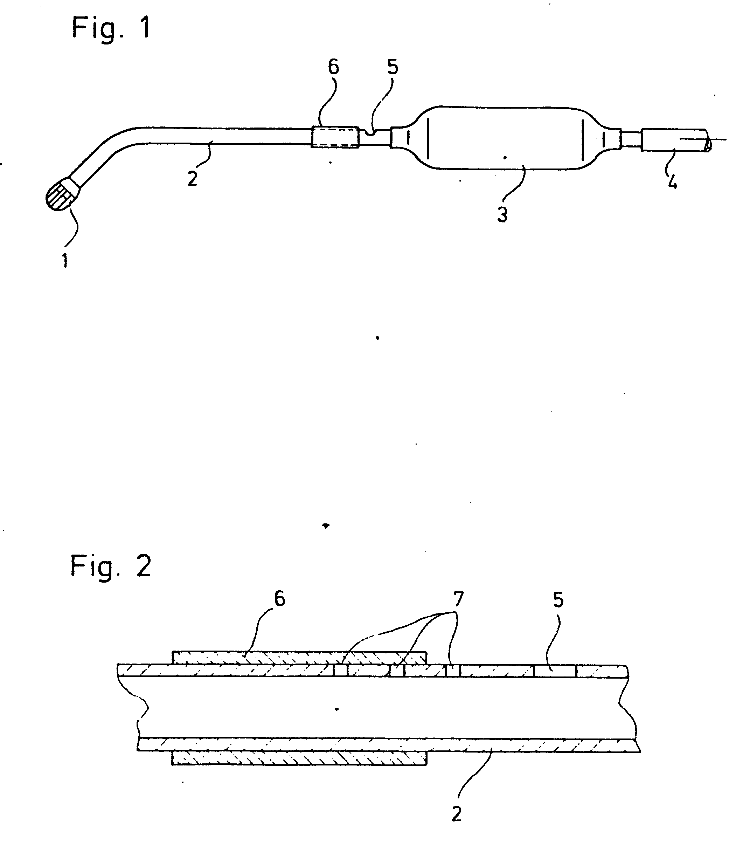

Die Erfindung wird anhand der Figuren 1 und 2 nachstehend erläutert.

Gemäß Figur 1 besteht ein erfindungsgemäßes Absaugrohr aus folgenden Teilen:

Dem Saugkorb (1), dem abgewinkelten Rohr (2) dem Handgriff (3), der Bohrung zur Sogregulierung (5) und der Einrichtung zur Unterdruckbegrenzung (6).
Über den Verbindungsschlauch (4) wird das Absaugrohr mit einer Vakuumquelle verbunden.
Die Strömungsrichtung ist durch den Pfeil dargestellt.

- 3 -

Selbstverständlich müssen an praktisch ausgeführten Absaugrohren nicht alle Teile in der
erfindungsgemäßen Ausführung vorhanden sein.

Die Einrichtung zur Unterdruckbegrenzung
besteht gemäß Figur 2 aus einer auf dem
Rohr (2) beweglichen Hülse (6) und mehreren
axial angeordneten Bohrungen ( 7 ).
Je nach Stellung der Hülse wird eine unterschiedliche Zahl von Bohrungen freigegeben
und somit Partialdrucke eingestellt. Durch
die einströmende Nebenluft wird der am Saugmund erreichbare Unterdruck auf das gewünschte
Maß reduziert. Die Berechnung des erreichten
Druckabfalls an einer bestimmten Bohrung erfolgt durch Umwandlung der allgemeinen Formel
für den Druckabfall im Rohrsystem

$$\Delta P = \lambda \frac{L \cdot 8}{d \cdot 2g} \cdot \omega^2$$

Die Bohrungen (7) können so angeordnet sein, daß
zum Verschließen, die Hülse ( 6 ) entweder axial
verschoben oder gedreht werden muß. Anstelle
der Bohrungen sind auch entsprechende Schlitze
denkbar. Zusätzlich kann das Absaugrohr noch mit
einer Querbohrung dieser bekannten Sogregulierung (5)
ausgestattet sein. Durch Verschließen durch Daumen
oder Zeigefinger kann noch eine bestimmte Luftmenge einströmen.

Die Hülse (6) kann einen geschlossenen Mantel aufweisen oder längsgeschlitzt ausgebildet sein. Wenn
die Bohrungen (7) beispielsweise auf einer Geraden
liegen, kann durch die längsgeschlitzte Hülse ein
gleichzeitiges Öffnen der Bohrungen (7) durch Drehen
erfolgen.

- 4 -

A n s p r ü c h e

1.   Medizinisches Absaugrohr zum Absaugen von Körperprodukten oder Spülflüssigkeiten bei Operationen, dessen eines Ende ein Mundstück trägt und dessen anderes Ende an eine Saugvorrichtung angeschlossen ist, dadurch gekennzeichnet, daß das Rohr (2) auf einem Teilstück seiner Länge eine sich axial erstreckende Wanddurchbrechung aufweist und auf dem Rohr (2) eine Hülse (6) beweglich angeordnet ist.

2.   Medizinisches Absaugrohr nach Anspruch 1, dadurch gekennzeichnet, daß die Hülse (6) auf dem Rohr (2) drehbar und/oder längsverschiebbar angeordnet ist.

3.   Medizinisches Absaugrohr nach Ansprüchen 1 und 2, dadurch gekennzeichnet, daß die Hülse einen geschlossenen Mantel aufweist.

4.   Medizinisches Absaugrohr nach Ansprüchen 1 und 2, dadurch gekennzeichnet, daß die Hülse längsgeschlitzt ausgebildet ist.

5.   Medizinisches Absaugrohr nach Ansprüchen 1 - 4, dadurch gekennzeichnet, daß die Wanddurchbrechung aus mehreren radialen Bohrungen (7) gebildet ist.

6.   Medizinisches Absaugrohr nach Anspruch 5, dadurch gekennzeichnet, daß die Bohrungen (7) auf einer Geraden liegen.

7.   Medizinisches Absaugrohr nach Anspruch 5, dadurch gekennzeichnet, daß die Bohrungen (7) auf dem Rohrumfang verteilt sind.

8.     Medizinisches Absaugrohr nach Ansprüchen 1 - 4, dadurch gekennzeichnet, daß die Wanddurchbrechung aus mindestens einem Schlitz gebildet ist.

9.     Medizinisches Absaugrohr nach Anspruch 8, dadurch gekennzeichnet, daß der Schlitz konisch gestaltet ist.

1o.     Medizinisches Absaugrohr nach Ansprüchen 1 - 9, dadurch gekennzeichnet, daß das Rohr (2) zusätzlich eine Fingerbohrung (5) aufweist.

11.     Medizinisches Absaugrohr nach Ansprüchen 1- 1o, dadurch gekennzeichnet, daß die Wanddurchbrechung nach folgender Formel angeordnet und ausgelegt ist:

$$\Delta p = \lambda \frac{L \cdot 8}{d \cdot 2g} \cdot \omega^2$$

Fig. 1

Fig. 2

0007602

Europäisches
Patentamt

**EUROPÄISCHER RECHERCHENBERICHT**

Nummer der Anmeldung

EP 79 10 2583

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>AU - A - 423 662</u> (JOHNSON & JOHNSON) <br><br> * Abbildung 1; Seite 2, Absatz 6; Seite 3, Absätze 1,2 * <br><br> -- | 1-3,5 |
| | <u>US - A - 3 713 443</u> (VERNITRON CORP) <br> * Anspruch 1 * <br><br> ---- | 1,5,7 |

**KLASSIFIKATION DER ANMELDUNG (Int.Cl. 3)**

A 61 M 1/00

**RECHERCHIERTE SACHGEBIETE (Int. Cl. 3)**

A 61 M

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 26-10-1979 | NOESEN |

EPA form 1503.1   06.78